# EUROPEAN PATENT APPLICATION

(11) **EP 1 949 873 A1**
(43) Date of publication of application: **30.07.2008**
(21) Application number: 06810437.1
(22) Date of filing: 22.09.2006
(51) Int. Cl.: A61F 2/74, A61F 2/58, A61H 1/02

(54) **MOTION ASSISTANCE APPARATUS AND METHOD OF ASSISTING MOTION**

(30) Priority: 11.10.2005 JP 2005296272
(71) Applicant: MATSUSHITA ELECTRIC INDUSTRIAL CO., LTD., Kadoma-shi Osaka 571-8501 (JP)
(72) Inventor: UEDA, Keisuke c/o Matsushita Electric Industrial Co., Ltd., 2-1-61 Shiromi, Chuo-ku Osaka 540-6207 (JP); FUJIMOTO, Hiromichi c/o Matsushita Electric Industrial Co., Ltd., 2-1-61 Shiromi, Chuo-ku Osaka 540-6207 (JP); SHIROGAUCHI, Go c/o Matsushita Electric Industrial Co., Ltd., 2-1-61 Shiromi, Chuo-ku Osaka 540-6207 (JP)
(74) Representative: Eisenführ, Speiser & Partner
(86) International application number: PCT/JP2006/318824
(87) International publication number: WO 2007/043308

(57) **Abstract**

A movement assisting device including a plurality of attachments to be attached over a joint of a user; an actuator suspended between the plurality of attachments; a sensor, attached to the user, for detecting movement of a muscle; and a control unit for controlling the actuator based on the movement of the muscle detected by the sensor is provided.

## Description

### TECHNICAL FIELD

The present invention relates to a movement assisting device and a movement assisting method for assisting the movement of elbow, wrist, knee, or the like of a human body using an actuator.

### BACKGROUND ART

Various training devices for regaining the muscle strength of each part of hands and legs, torso, neck or the like have been conventionally developed for patients having disability in motor function.

Various devices such as walk assisting device, stair elevating lift device, or carrying lift device for assisting daily movement of an elderly who has lost physical strength, and alleviating physical strain on the caretaker have been developed in an aim of aiding muscle strength.

In such devices, a technique for aiding the muscle strength of the user by being worn is proposed in Japanese Laid-Open Patent Publication No. 2001-286519 and Japanese Laid-Open Patent Publication No. 2001-276101.

First, the device disclosed in Japanese Laid-Open Patent Publication No. 2001-286519 includes an actuator for providing an aiding force in the bending direction to the joint portion of the user, a control means for controlling the movement of the actuator, and a pair of attachments attached to both sides of the actuator. According to such technique, the bending and extending movements of the joint can be assisted by operating the actuator.

The device disclosed in Japanese Laid-Open Patent Publication No. 2001-276101 includes an attachment having flexibility, being of cylindrical shape, and being closely attached while wrapping the joint of the user; and an actuator integrated at the outer periphery of the attachment. According to such technique as well, the bending and extending movements of the joint can be assisted by operating the actuator and bending the attachment.

In the prior art, however, the movement can only be assisted in the direction and with the force instructed by the control means or defined in advance, when assisting the bend and the extension of the joint of the user. Thus, the muscle or the joint of the user might get injured if the movement is assisted in a direction different from the direction desired by the user or if the movement is assisted with a force different from the force desired by the user such as when the force is too strong, or the effect of aiding the muscle strength might lower if the force is too weak.

### DISCLOSURE OF THE INVENTION

In view of the above issues, it is an object of the present invention to provide a movement assisting device and a movement assisting method capable of assisting the movement in a direction and with a force desired by the user when assisting the movement of the user.

A movement assisting device of the present invention includes a plurality of attachments to be attached over a joint of a user; an actuator suspended between the plurality of attachments; a sensor, attached to the user, for detecting movement of a muscle; and a control unit for controlling the actuator based on the movement of the muscle detected by the sensor.

According to such configuration, since the control unit controls the actuator based on the movement of the muscle detected by the sensor, the intension of movement of the user can be reflected on the movement assisting device, and the movement can be assisted in the direction and with the force desired by the user. Furthermore, the movement can be assisted without inhibiting the movement of the joint of the user since the actuator nor the attachment is not applied to the joint.

The control unit may generate a force to aid the movement of the muscle at the actuator based on the movement of the muscle detected by the sensor.

According to such configuration, a movement assisting device suited for rehabilitation application with respect to a person with weak muscle strength can be realized.

A pneumatic source for supplying air to the actuator may be further arranged; wherein the actuator is a pneumatic rubber artificial muscle; and the control unit operates the actuator by controlling air pressure of the pneumatic rubber artificial muscle.

According to such configuration, a configuration having a low possibility of contacting the human body and giving an unpleasant feeling is realized since a rubber artificial muscle is used as the actuator. Even if attached to the distal end portions of the four limbs, the moment on the base of the four limbs becomes small and the load on the human body reduces since the rubber artificial muscle serving as the actuator is light. Furthermore, since the movement range is defined by the contraction limit of the rubber artificial muscle, the range of motion of the joint of the user is not exceeded and excessive load is not applied to the joint.

The plurality of actuators may be suspended between a pair of attachments; and the plurality of actuators may be arranged at positions antagonizing each other with respect to the joint.

According to such configuration, different movements to each other such as bending and extending can be assisted with respect to one joint.

A pair of actuators may be arranged between the pair of attachments.

According to such configuration, the movement can be smoothly assisted in a balanced manner. The perpendicular relationship between the operation axis of the joint and the operation axis of the actuator is prevented from shifting. If four or more actuators are arranged in an antagonizing manner with respect to the joint, the rotation movement can also be assisted in addition to bending and extending of the joint.

A joint member of an elastic body may be arranged between the attachments.

According to such configuration, the rotation movement can also be assisted in addition to bending and extending of the joint.

The sensor may be attached to a predetermined site of the user; and the actuator may be attached to assist the movement of the predetermined site of the user.

According to such configuration, the movement of the muscle of the user can be detected, and the detected movement of the muscle can be assisted.

The sensor may be attached at one site of the user; the actuator may be arranged to assist the movement of another site of the user; and the one site and the another site may be symmetric sites.

According to such configuration, the sensor is attached to the site on the healthy side of the user, and the actuator is attached to the site of weak muscle strength of the user, so that a more appropriate rehabilitation can be performed on the user whose muscle strength of one half of the body is weak by moving both the healthy side and the side of weak muscle strength.

The sensors may be arranged in plurals, where one of the sensors of the plurality of sensors may be attached to the another site of the user.

According to such configuration, the movement of the site of weak muscle strength of the user can be detected, whereby the regaining degree of the muscle strength of the user can be known.

The sensor may be attached to a predetermined site of an indicator who gives instructions to the user; and the actuator may be attached to the predetermined site of the user.

According to such configuration, the user can more easily perform the movement according to the movement of the indicator, and an effective rehabilitation can be performed.

The actuator may be arranged in plurals; and the plurality of actuators may be attached at to the predetermined position of each of a plurality of users.

According to such configuration, a plurality of users can receive movement assistance by the movement of the indicator.

The control unit may control the actuator, based on the movement of the muscle detected by the sensor, so as to become a load with respect to the movement of the muscle.

According to such configuration, applications to training applications such as exercise are possible in addition to rehabilitation applications.

A movement assisting method of the present invention relates to a movement assisting method using a movement assisting device including a plurality of attachments to be attached over a joint of a user; an actuator suspended between the plurality of attachments; and a sensor, attached to the user, for detecting movement of a muscle; the method including the step of controlling the actuator based on the movement of the muscle detected by the sensor.

According to such method, the movement can be assisted in the direction and with the force desired by the user since the actuator is controlled based on the movement of the muscle detected by the sensor.

### BRIEF DESCRIPTION OF THE DRAWINGS

Fig. 1 is a perspective view showing a state in which a movement assisting device according to a first embodiment of the present invention is attached to the arm.
Fig. 2 is a perspective view of the movement assisting device in the state shown in Fig. 1 according to the first embodiment of the present invention seen from the rear side.
Fig. 3 is a perspective view of an actuator unit portion of the movement assisting device according to the first embodiment of the present invention.
Fig. 4 is a functional block diagram of the movement assisting device according to the first embodiment of the present invention.
Fig. 5 is a view showing a state in which the elbow of the user is bent by the movement assisting device according to the first embodiment of the present invention.
Fig. 6 is a flowchart showing the operation steps when the movement assisting device according to the first embodiment of the present invention assists the movement of the user.
Fig. 7 is a view describing the function of a control unit of the movement assisting device according to the first embodiment of the present invention.
Fig. 8 is a view showing a configuration of a movement assisting device according to a second embodiment of the present invention.
Fig. 9 is a view showing another example of the movement assisting device according to the second embodiment of the present invention.
Fig. 10 is a view showing a configuration of a movement assisting device according to a third embodiment of the present invention.

### Description of Symbols

1, 2, 3 cuff (attachment)
4 to 11 rubber artificial muscle (actuator)
12 to 15, 22 to 25 sensor
16, 66, 76, 86 control unit
17, 18 joint member
22 pneumatic source
50, 60, 70, 80 movement assisting device
52, 62, 72, 82 sensor unit
54, 64, 74, 84, 85 actuator unit

### BEST MODE FOR CARRYING OUT THE INVENTION

Embodiments of the present invention will now be described with reference to the drawings.

### (First Embodiment)

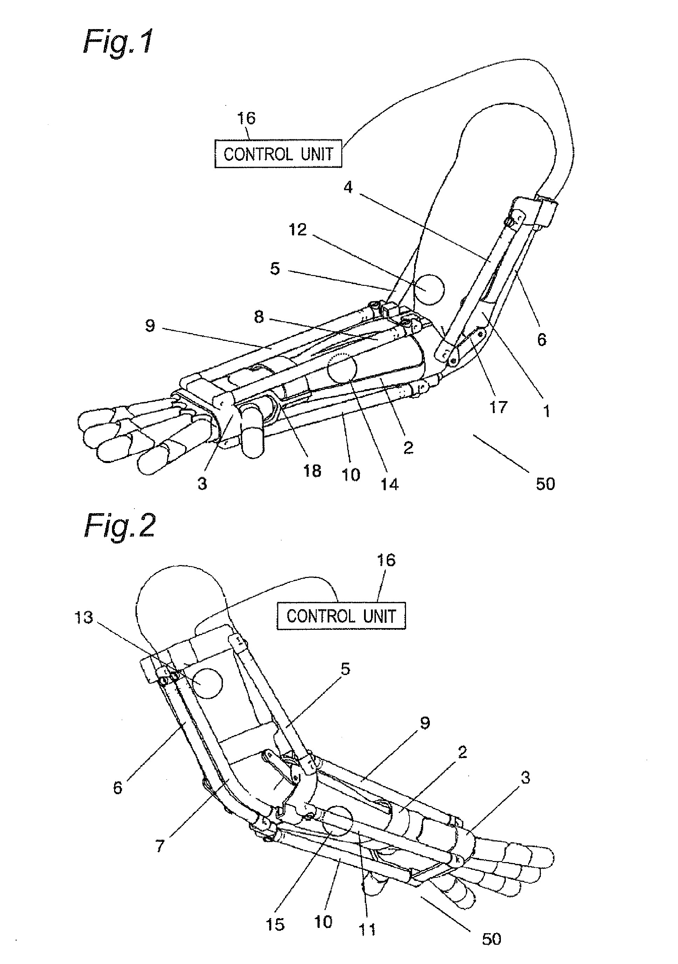

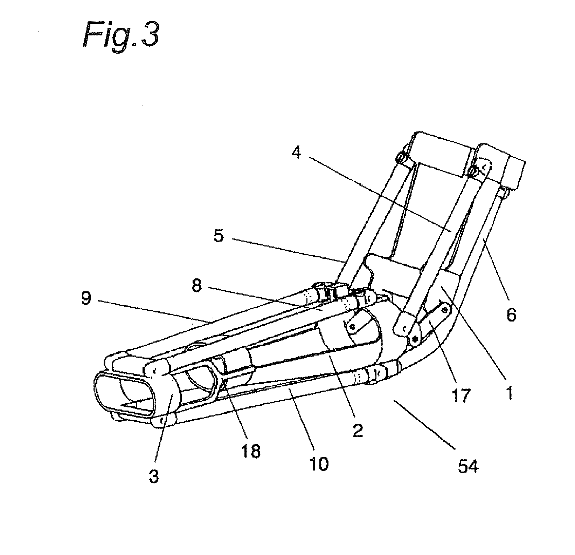

Fig. 1 is a perspective view showing a state in which a movement assisting device 50 according to a first embodiment of the present invention is attached to the arm, and Fig. 2 is a perspective view of the movement assisting device 50 in the state shown in Fig. 1 seen from the rear side. Fig. 3 is a perspective view of an actuator unit 54 portion of the movement assisting device 50.

As shown in Fig. 1 to Fig. 3, the movement assisting device 50 according to the first embodiment of the present invention is an attachment arranged across the elbow joint of the user, and includes a cuff 1 attached to the upper arm and a cuff 2 attached to the forearm, a rubber artificial muscle 4 and a rubber artificial muscle 5 arranged in pairs while being suspended between the cuff 1 and the cuff 2 to aid the movement in the bending direction of the elbow, as well as, a rubber artificial muscle 6 and a rubber artificial muscle 7 arranged in pairs at a position where the force generated by the rubber artificial muscle 4 and the rubber artificial muscle 5 can be canceled out (hereinafter referred to as "position of antagonizing") to aid the movement in the extending direction of the elbow.

The movement assisting device 50 also includes a cuff 3 attached at a position of a palm across the joint of the wrist with respect to the cuff 2 attached to the forearm, a rubber artificial muscle 8 and a rubber artificial muscle 9 as actuators for aiding the movement in the palmar flexion direction of the wrist arranged between the cuff 2 and the cuff 3, and a rubber artificial muscle 10 and a rubber artificial muscle 11 arranged to aid the movement in the dorsal flexion direction of the wrist at a position of antagonizing with the rubber artificial muscle 8 and the rubber artificial muscle 9.

The rubber artificial muscles 4 to 11 may be a McKibben pneumatic actuator including a cylinder part with an air valve and a sleeve part, also referred to as McKibben pneumatic rubber artificial muscle. When the McKibben pneumatic actuator is used, the actuator is contracted through the operation of the sleeve part by pressurizing and expanding the cylinder part through the air valve, and the actuator is extended by depressurizing the cylinder part from the above state.

The movement assisting device 50 further includes a joint member 17 arranged between the cuff 1 and the cuff 2, and a joint member 18 arranged between the cuff 2 and the cuff 3. Since the joint member 17 and the joint member 18 are respectively configured using an elastic body in the movement assisting device 50, the twisting (rotating) movement can also be assisted in addition to the bending and extending movement at each joint of the user.

The rubber artificial muscles 4 to 11, the cuffs 1 to 3, and the joint members 17, 18 are generically termed as an actuator unit 54.

The movement assisting device 50 further includes a sensor 12 arranged on the front side of the upper arm of the user to detect the movement of the biceps muscle of the arm, a sensor 13 arranged on the back side of the upper arm to detect the movement of triceps muscle of the arm, a sensor 14 arranged on the front side of the forearm of the user, a sensor 15 arranged on the back side of the forearm, and a control unit 16 for detecting the output of the sensors 12 to 15 and controlling the rubber artificial muscles 4 to 11 so as to assist the movement of the user according to the output.

The sensors 12 to 15 merely need to be a sensor that can measure the muscle strength of the user and various known sensors such as strain gauge can be used.

The sensors 12 to 15 are generically termed as a sensor unit 52.

The function of the control unit 16 may be realized by hardware of a dedicated circuit, or the function may be realized by describing a program for realizing the control steps to be hereinafter described and executing the same in a computer.

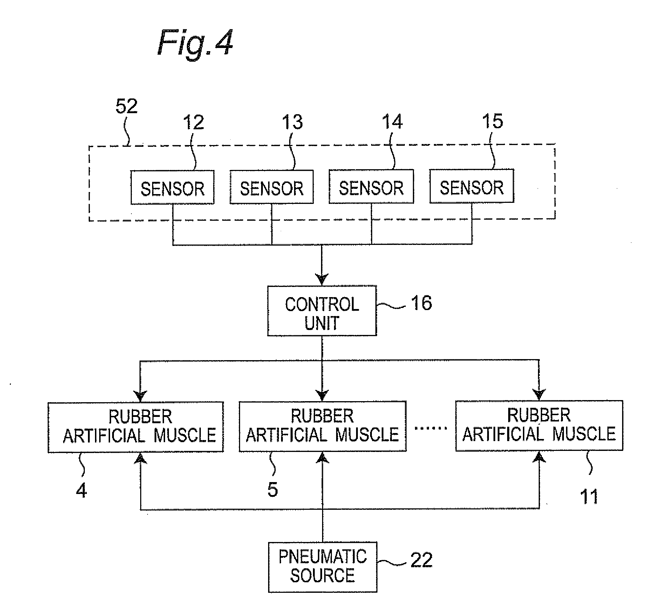

The function of the movement assisting device 50 will be described in detail below. Fig. 4 is a functional block diagram of the movement assisting device 50 according to the first embodiment of the present invention. As shown in Fig. 4, the sensors 12 to 15 of the sensor unit 52 are respectively connected to the control unit 16 in the movement assisting device 50. The control unit 16 detects the output of each sensor 12 to 15, determines what movement the user is trying to perform, and open/close controls the air valve of each connected rubber artificial muscle 4 to 11 of the pneumatic source 22 to change the air pressure based on the result, thereby operating each rubber artificial muscle 4 to 11 and performing movement assistance in the desired direction and with the desired force on the desired site of the user.

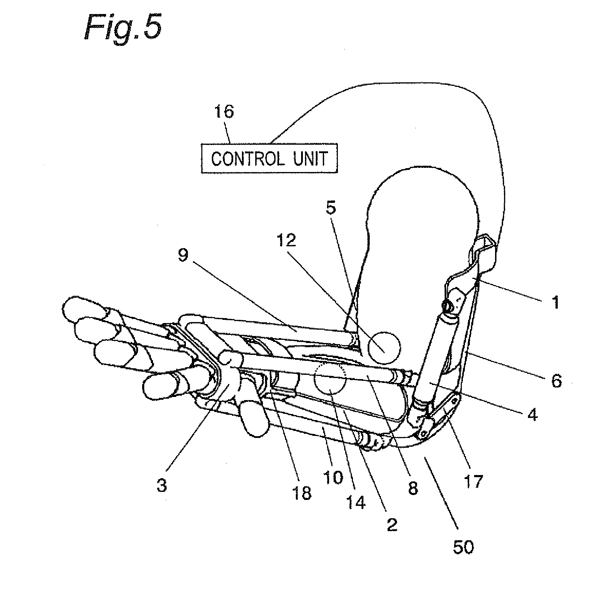

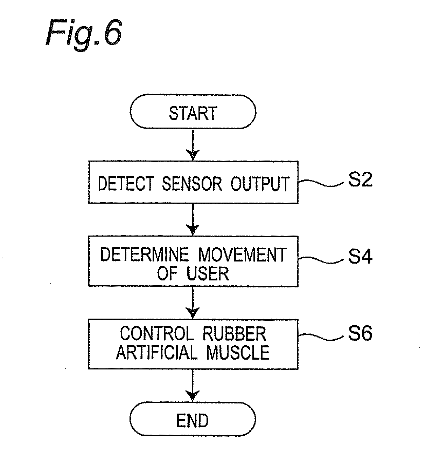

The movement assisting method of the user by the movement assisting device 50 will be further described in detail. Fig. 5 is a view showing a state in which the elbow of the user is bent by the movement assisting device 50 according to the first embodiment of the present invention. Fig. 6 is a flowchart showing operation steps when the movement assisting device assists the movement of the user.

First, the control unit 16 of the movement assisting device 50 starts to detect the output from the sensors 12 to 15, as shown in Fig. 6 (step S2).

When the control unit 16 detects the output from the sensors 12 to 15 in step S2, the control unit 16 determines which joint the user is trying to move and in which direction from the output (S4).

The control unit 16 causes the rubber artificial muscles 4 to 11 to perform the desired operation by open/close controlling the air valve of the rubber artificial muscles 4 to 11 (S6), as described above. The control unit 16 then returns to step S2, and again starts the detection of the sensors 12 to 15.

For instance, when the control unit 16 detects the output of the sensors 12 to 15 and determines that the user is trying to "bend the elbow joint" in step S4, the control unit 16 controls the air valve of the rubber artificial muscle 4 and the rubber artificial muscle 5 and pressurizes the inside of the respective cylinder thereby contracting the rubber artificial muscle 4 and the rubber artificial muscle 5 forming a pair and applying the biasing force in the pulling direction between the cuff 3 and the cuff 2 to apply a force for aiding the bend of the elbow joint of the user, as shown in Fig. 5.

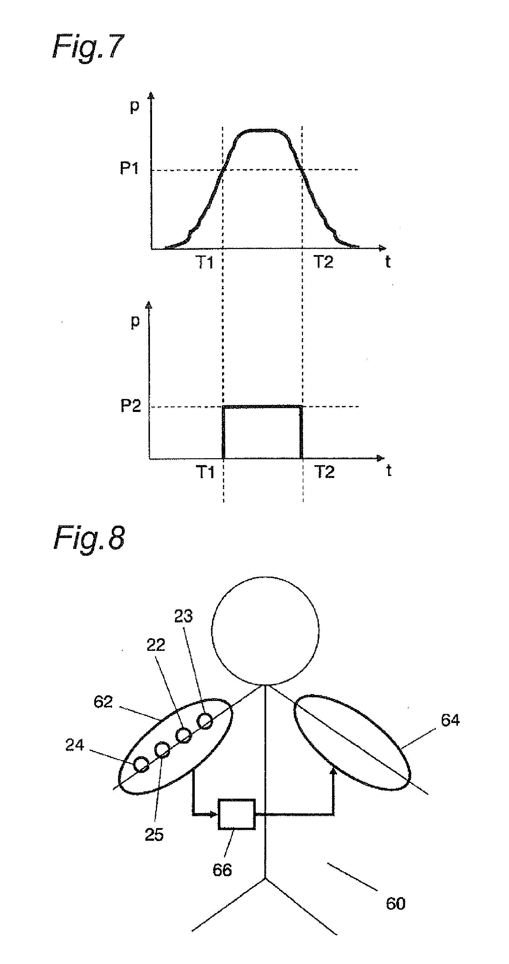

Fig. 7 is a view for describing the function of the control unit 16 of the movement assisting device 50 in the first embodiment of the present invention. In Fig. 7, as an example, a graph for the movement of the biceps muscle of the arm of the user detected from the sensor 12 is shown on the upper side, and a graph for the force to be applied on the rubber artificial muscles 4, 5 by the control unit 16 at the relevant moment is shown on the lower side. Each graph shows the magnitude P of the force on the vertical axis, and the time t on the horizontal axis.

For instance, when the control unit 16 detects the bending movement of the biceps muscle of the arm as shown on the graph on the upper side of Fig. 7, the control unit 16 controls the air valve of the rubber artificial muscle 4 and the rubber artificial muscle 5 so as to apply the biasing force P2 in the contracting direction as shown on the graph on the lower side of Fig. 7 on the rubber artificial muscle 4 and the rubber artificial muscle 5 when the detected force exceeds a predetermined threshold value P1 (time T1). The biasing force P2 in the contracting direction can then be applied on the biceps muscle of the arm of the user. On the other hand, the control unit 16 releases the biasing force P2 when the detected force underruns the predetermined threshold value P1 (time T2). An unintended force from the movement assisting device 50 is thereby prevented from being applied when the user relaxes the muscle.

The control unit 16 does not necessarily need to apply the biasing force P2 simultaneously with when the force detected by the sensor 12 exceeds the predetermined threshold value P1 (time T1). The user sometimes feel a sense of safety if there is a slight delay in the timing of applying the biasing force P2, and thus the delay amount is desirably adjustable. For instance, an adjustment unit such as switch or dial for adjusting the delay amount (specifically, shift amount between time T1 at when the force detected by the sensor 12 exceeds a predetermined threshold value P1 and time of applying the biasing force P2) may be arranged, so that the user can be movement assisted while feeling the most sense of safety by operating the adjustment unit.

For practical purposes, the relationship between the predetermined threshold value P1 and the biasing force P2 is
P1 > P2
whereby movement assistance can be performed without being aware by the user.

An excess load on the user can be prevented by providing an upper limit to the biasing force P2 and a movable range of the rubber artificial muscle used in the movement assistance.

The biasing force P2 used in the movement assistance may change with time. For instance, the biasing force P2 is set large at the start of rehabilitation, the biasing force P2 is made small with time, and the biasing force P2 is again set large at the termination of rehabilitation to perform warming up and cooling down thereby realizing a configuration of higher safety to the user.

The movement assisting device 50 simultaneously contracts the rubber artificial muscle 4 and the rubber artificial muscle 5 by the same length to maintain a perpendicular relationship between the operation axis of the joint of the user and the operation axes of the rubber artificial muscle 4 and the rubber artificial muscle 5. The output of the rubber artificial is thereby efficiently transmitted to the muscle of the user, and the load on the joint of the user caused by the shift of the operation axis of the joint of the user and the operation axes of the rubber artificial muscles from perpendicularity can be reduced.

When the control unit 16 determines that the user is trying to extend the elbow in step S4 of Fig. 6, the control unit 16 contracts the rubber artificial muscle 6 and the rubber artificial muscle 7 in step S6 to assisting the extending movement of the elbow. Furthermore, similar to the movement assistance the elbow, the movement assistance of palmar flexion is realized by contracting the rubber artificial muscle 8 and the rubber artificial muscle 9, and movement assistance of dorsal flexion is realized by contracting the rubber artificial muscle 10 and the rubber artificial muscle 11 in the movement assistance of the wrist.

Furthermore, when the control unit 16 determines that the user is trying to rotate the elbow in step S4, the control unit 16 performs a control to contract the rubber artificial muscle 4, the rubber artificial muscle 9, and the rubber artificial muscle 10 in step S6 to movement assist inner rotation of the forearm. Furthermore, the control unit 16 can contract the rubber artificial muscle 5, the rubber artificial muscle 8, and the rubber artificial muscle 11 to movement assist outer rotation of the forearm. In the movement assisting device 50, the twisting movement at the joint is facilitated since the joint member 17 and the joint member 18 of an elastic body are used to connect the cuffs 1 to 3, as described above.

As described above, the movement in the direction and with the force desired by the user can be appropriately assisted in the movement assisting device according to the first embodiment of the present invention since the control unit detects the signal output from the sensor, determines what movement the user is trying to perform, and operates the rubber artificial muscle based on the determination result.

The movement assisting device 50 does not include components that may be sandwiched on the inner side of the joint, and thus the joint can be bent in large amount of angle, and application can be developed to tasks of carrying heavy object, that is conveying loads in factory, mover, and the like.

The user hardly feels pain, and application can be developed to medical and care fields such as carrying patients since a soft pneumatic rubber artificial muscle is used as an actuator in the movement assisting device 50.

### (Second Embodiment)

A movement assisting device 60 according to a second embodiment of the present invention will now be described. Fig. 8 is a view showing a configuration of the movement assisting device 60.

As shown in Fig. 8, the movement assisting device 60 includes a sensor unit 62, an actuator unit 64, and a control unit 66. The movement assisting device 60 differs from the movement assisting device 50 in the first embodiment in that the sensor unit 62 is attached to an arm opposite to the arm attached with the actuator unit 64.

According to such configuration, the movement assisting device 60 enables the control unit 66 to detect the movement of the arm attached with the sensor unit 62, and operate the other arm with the rubber artificial muscle of the actuator unit 64 so as to assist the relevant movement.

According to such configuration, rehabilitation effective for users who have become paralyzed on one side of the body can be performed by using the movement assisting device 60. For instance, if the left arm of the user is paralyzed, the sensor unit 62 is attached to the arm (right arm) on the healthy side that is not paralyzed, and the actuator unit 64 is attached to the arm (left arm) on the paralyzed side. At this point, the sensor 22 is arranged on the surface side of the upper arm of the right arm of the user, the sensor 23 is arranged on the back side of the right arm of the user, the sensor 24 is arranged on the front side of the forearm of the right arm of the user, and the sensor 25 is arranged on the back side of the forearm of the right arm of the user.

According to such configuration, the user himself/herself can move the arm on the healthy side, so that the control unit 16 detects the movement of muscle of the healthy arm through the sensor unit 62, and based on such movement, assists the movement of the corresponding muscle of the arm on the paralyzed side by the actuator unit 64. In other words, the user can move the arm on the paralyzed side with willingness and perform rehabilitation while holding an image of recovery by trying to move both arms in a similar manner while looking at the arm on the healthy side using the movement assisting device 60. It is described in document 1 ("NOU NI MARAKASU NO AME GA FURU" written by Shinichiro Kurimoto released June 26, 2000 from Kobunsha Publishing Co. Ltd), and document 2 ("Example of cerebral stroke paralysis in which upper limb function improved by Constrained-induced movement therapy" Jpn J Rehabil Med, Vol 40, No. 12, 2003) that such rehabilitation reduces time required for recovery and is very effective on patients who are paralyzed on one side of the body as it can be performed over a relatively long period of time by the user himself/herself without being instructed by the work therapist etc.

Therefore, through the use of the movement assisting device 60 according to the second embodiment of the present invention, the muscle strength can be unconsciously generated at the muscle on the paralyzed side thereby enhancing not only the muscle strength but also the sense of balance and the neural network.

In the movement assisting device 60, an example of attaching the sensor unit 62 to the arm on the healthy side and attaching the actuator unit 64 on the arm on the paralyzed side has been described, but the present invention is not limited to such example. In addition to the above described configuration, for example, the sensor unit may also be attached to the arm on the parlayed side, so that the control unit 66 can detect the movement of the muscle of on the paralyzed side and measure the regaining degree of the muscle strength of the user.

In this case, the control unit 66 measures the regaining degree of the muscle strength of the user and changes the amount of assisting the movement (e.g., reduce assisting amount the more the muscle strength regains) according to the result, so that a more effective rehabilitation can be carried out.

A grip, and the like may be gripped by the arm on the side attached with the sensor unit 62 and a pressure sensor, and the like may be arranged on the grip so that the grip strength of the user can be detected, where a safety switch may operate according to the change in the grip strength (e.g., safety switch operates to turn OFF the device when the grip strength lowers), whereby a configuration excelling in safety can be realized, and a more effective rehabilitation can be performed since the muscle strength of the arm with more strength is easier to sense.

In the present embodiment, an example of causing the actuator unit 64 to perform the same movement as detected by the sensor unit 62 has been described, but the present invention is not limited to such example. For instance, the actuator unit 64 may perform the movement of opposite phase as the movement detected by the sensor unit 62 (e.g., when the sensor unit 62 detects bending of the elbow, the actuator unit is caused to extend the elbow). When such movement is performed, the user can feel enjoyment as if playing drums (as if stepping when attached to the legs).

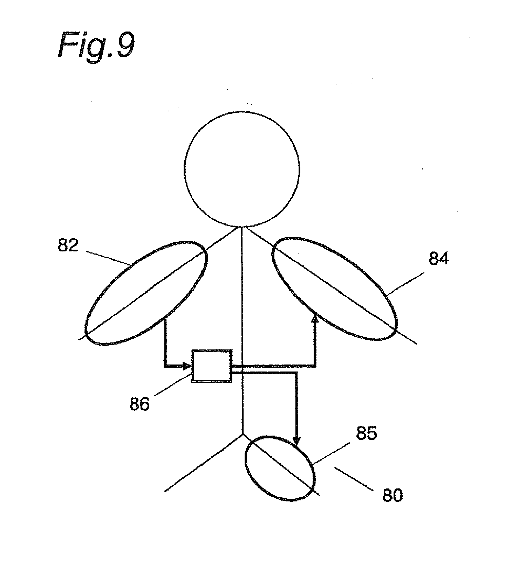

Furthermore, as in another example of a movement assisting device 80 according to the second embodiment of the present invention shown in Fig. 9, a sensor unit 82 is attached to one arm of the user, so that a control unit 86 detects the output thereof, and causes an actuator unit 84 attached to the other arm of the user and an actuator unit 85 attached to one of the legs of the user to perform movement assistance based on the output.

In this case as well, the control unit 86 causes the actuator units 84 and 85 to perform the movement of opposite phase as the movement detected by the sensor unit 82. According to such configuration, when the user walks while being conscious of swinging one arm on the healthy side, the other arm and the legs are movement assisted and walking rehabilitation can be performed while holding an image of walking naturally.

Similar effects are obtained if the sensor unit 82 is attached to one leg and the actuator units 84 and 85 are attached to the other leg and one of the arms.

### (Third Embodiment)

A movement assisting device 70 according to a third embodiment of the present invention will now be described.

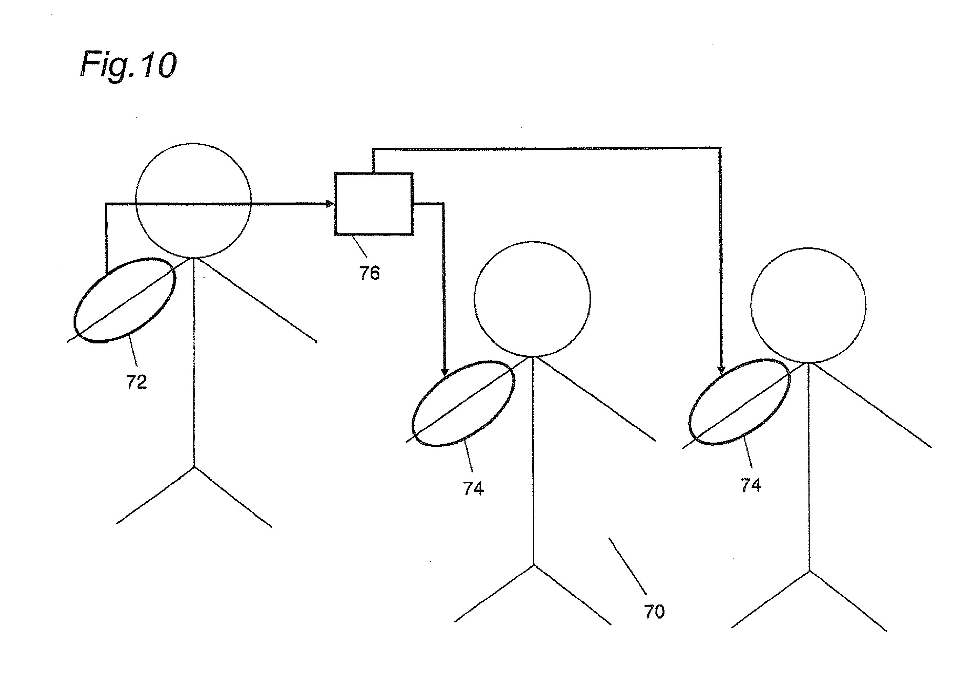

Fig. 10 is a view showing a configuration of the movement assisting device 70 according to the third embodiment of the present invention.

As shown in Fig. 10, the movement assisting device 70 includes a sensor unit 72, an actuator unit 74, and a control unit 76 connected to the sensor unit 72 and the actuator unit 74.

The movement assisting device 70 differs the most from the movement assisting device 50 according to the first embodiment and the movement assisting device 60 according to the second embodiment in that the sensor unit 72 and the actuator unit 74 are attached to different people. In the present embodiment, an example where the movement assisting device 70 includes a plurality of actuator units 74 with respect to one sensor unit 72 is shown.

The sensor unit 72 of the movement assisting device 70 is attached to an arbitrary site (e.g., right arm) of a person (hereinafter referred to as indicator) who gives instructions to the user such as work therapist or an instructor, and the actuator unit 74 is attached to the user at the site same as the site of the indicator attached with the sensor unit 72.

The control unit 76 detects the output from the sensor unit 72 attached to the indicator, determines how the muscle of which site of the indicator moves, and operates the rubber artificial muscle of the actuator unit 74 attached to the user based on the result. When a plurality of actuator units 74 is arranged, the control unit 76 performs the control on the plurality of actuator units 74.

The user can easily move according to the movement of the indicator by using the movement assisting device 70. This is because the movement is assisted so that the muscle of the user corresponding to the movement of the indicator moves in a similar manner by the movement assisting device 70.

When the movement assisting device 70 includes the plurality of actuator units 74, the indicator can give instruction of motion to a plurality of users at the same time, and furthermore, the muscle of each of the plurality of users is movement assisted to perform the same movement as the movement of the indicator, whereby the device is very effective as a device for assisting rehabilitation in hospitals, rehabilitation institutions, and the like. In this case, the motivation of the user can be further enhanced since the plurality of users can undergo rehabilitation at the same time.

In the movement assisting device according to the embodiment of the present invention, a case of using the rubber artificial muscle as the actuator has been described, but the present invention is not limited to such example. For instance, similar effects are obtained by realizing the function of the actuator with a combination of a motor and a wire. In this case, a power supply is used as a drive source and the control unit performs rotation control of the motor to realize the function of the movement assisting device of the present invention.

In the movement assisting device according to the embodiment of the present invention, the rubber artificial muscle used in assisting bending and extending movements of each joint is arranged by twos, but the present invention is not limited to such example. A configuration in which one rubber artificial muscle is arranged, and a configuration in which three or more rubber artificial muscles are arranged in the direction of assisting the movement of each joint may be adopted.

In the movement assisting device according to the embodiment of the present invention, a configuration of using the strain gauge as the sensor is described but the present invention is not limited to such configuration. As long as the sensor can detect the movement of the muscle, any type of sensor can be used, and various sensors such as position sensor, force sensor, torque sensor, gravity sensor, gravitational acceleration sensor, speed sensor, acceleration sensor, angle sensor, angular speed sensor, voice sensor, muscle potential sensor, displacement sensor, pressure sensor, pneumatic sensor, brain wave sensor, flow rate sensor, temperature sensor, humidity sensor, static electricity sensor, infrared sensor, photo-electronic sensor, vibration sensor, impact sensor, current sensor, voltage sensor, magnetic sensor, ultrasonic wave sensor, or the like may be used.

In the movement assisting device according to the embodiment of the present invention, description is made with the attachment as a cuff, but the present invention is not limited to such configuration. For instance, a combination of clothes and cuff can be used as the attachment so that the movement assisting device can be attached as if wearing clothes, whereby a configuration that can be attached by the user without resistance is realized. In this case, the cuff and the clothes are made detachable so that the clothes portion can be freely washed, whereby a configuration excelling in practicability is realized.

Injuries in joint caused by exercising under a cold environment can be prevented by embedding a heater in the attachment. In this case, it is practicable to turn ON/OFF the heater according to outside air temperature in a configuration equipped with the temperature sensor.

In the movement assisting device according to the embodiment of the present invention, an example in which the control unit operates the actuator so as to aid the movement of the muscle detected by the sensor has been described, but the present invention is not limited to such example. For instance, the control unit can apply a force on the actuator in a direction that becomes a load (e.g., direction of extending the elbow when the sensor detects bending of the elbow) with respect to the movement of the muscle detected by the sensor, whereby application to not only the rehabilitation application but also to sports fields such as exercise and training also becomes possible.

Furthermore, in the movement assisting device according to the embodiment of the present invention, description is made with the site to be attached being the arm, but the present invention is not limited to such example. It can be attached to any site of the human body other than the arm as long as joints are present such as fingers of hands and legs, knee, ankle, hip joint, hip, neck, shoulder, and the like.

### INDUSTRIAL APPLICABILITY

Therefore, a significant effect of performing movement assistance in the direction and with the force desired by the user is obtained according to the present invention, whereby it is effective as the movement assisting device and the movement assisting method for assisting the movement of the elbow, wrist, knee, or the like of the human body.

## Claims

1. A movement assisting device comprising:
a plurality of attachments to be attached over a joint of a user;
an actuator suspended between the plurality of attachments;
a sensor, attached to the user, for detecting movement of a muscle; and
a control unit for controlling the actuator based on the movement of the muscle detected by the sensor.

2. The movement assisting device according to claim 1, wherein the control unit generates a force to aid the movement of the muscle at the actuator based on the movement of the muscle detected by the sensor.

3. The movement assisting device according to claim 2, further comprising a pneumatic source for supplying air to the actuator; wherein
the actuator is a pneumatic rubber artificial muscle; and
the control unit operates the actuator by controlling air pressure of the pneumatic rubber artificial muscle.

4. The operation assisting device according to claim 3, wherein
the plurality of actuators is suspended between a pair of attachments; and
the plurality of actuators is arranged at positions antagonizing each other with respect to the joint.

5. The operation assisting device according to claim 4, wherein a pair of actuators are arranged between the pair of attachments.

6. The operation assisting device according to claim 2, wherein a joint member made of an elastic body is arranged between the attachments.

7. The operation assisting device according to claim 2, wherein
the sensor is attached to a predetermined site of the user; and
the actuator is attached to assist the movement of the predetermined site of the user.

8. The operation assisting device according to claim 2, wherein
the sensor is attached at one site of the user;
the actuator is arranged to assist the movement of another site of the user; and
the one site and the another site are symmetric sites.

9. The operation assisting device according to claim 8, wherein
the sensor is arranged in plurals; and
one of the sensors of the plurality of sensors is attached to the another site of the user.

10. The operation assisting device according to claim 2, wherein
the sensor is attached to a predetermined site of an indicator who gives instructions to the user; and
the actuator is attached to the predetermined site of the user.

11. The operation assisting device according to claim 10, wherein
the actuator is arranged in plurals; and
the plurality of actuators is attached to the predetermined position of each of a plurality of users.

12. The operation assisting device according to claim 1, wherein the control unit controls the actuator, based on the movement of the muscle detected by the sensor, so as to become a load on the movement of the muscle.

13. A movement assisting method using a movement assisting device including a plurality of attachments to be attached over a joint of a user; an actuator suspended between the plurality of attachments; and a sensor, attached to the user, for detecting movement of a muscle; the method comprising the step of:
controlling the actuator based on the movement of the muscle detected by the sensor.
